# FASCICULE DE BREVET EUROPEEN

(11) **EP 0 802 769 B1**
(45) Date de publication et mention de la délivrance du brevet: **29.12.1999**
(21) Numéro de dépôt: 95940332.0
(22) Date de dépôt: 17.11.1995
(51) Int. Cl.: A61B 10/00

(54) **DISPOSITIF A USAGE UNIQUE DE TRANSFERT D'UN LIQUIDE ACTIF DANS UNE CAVITE INTRACORPORELLE**
EINMALVORRICHTUNG ZUM EINBRINGEN EINER AKTIVEN FLÜSSIGKEIT IN EINE KÖRPERHÖHLE
SINGLE-USE DEVICE FOR DELIVERING AN ACTIVE LIQUID INTO A BODY CAVITY

(30) Priorité: 21.11.1994 FR 9414249
(43) Date de publication de la demande: 29.10.1997
(73) Titulaire: Chaffringeon, Bernard, 1025 Saint-Sulpice (CH)
(72) Inventeur: SGRO, Jean, Claude, F-21000 Dijon (FR)
(74) Mandataire: Guerre, Dominique
(86) Numéro de dépôt international: FR9501521
(87) Numéro de publication internationale: WO9615724

(56) Documents cités:
- WO-A-80/01353
- WO-A-83/01741
- FR-A- 2 353 640
- US-A- 3 519 364
- US-A- 4 257 427
- US-A- 4 317 454
- US-A- 5 357 977

## Description

La présente invention concerne le transfert ou circulation d'un liquide actif dans/ou à l'intérieur d'une cavité intracorporelle du corps humain ou d'un animal, notamment au contact de la muqueuse de ladite cavité.

Par cavité intracorporelle, on entend toute cavité du corps, notamment allongée, à laquelle on peut accéder de manière externe à différentes fins, notamment cliniques, thérapeutiques, prophylactiques, ou de diagnostic, mais aussi à des fins cosmétiques ou d'hygiène corporelle. A titre d'exemple d'une telle cavité, on peut citer le vagin de la femme qui s'étend de la vulve jusqu'au col utérin, dans lequel on désire transférer ou faire circuler un liquide actif se mélangeant à la glaire cervicale par exemple.

En conséquence, les liquides actifs considérés par la présente invention sont de manière générale des liquides ou fluides de traitement, comprenant aussi bien un agent liquide de mouillage, solubilisant, ou fluidifiant, notamment d'un liquide ou fluide corporel présent dans la cavité intracorporelle considérée, qu'un agent thérapeutique, prophylactique, ou de diagnostic, ou encore qu'un agent cosmétique ou d'hygiène corporelle, ou finalement qu'un agent antiseptique, bactéricide, fongicide, ou spermicide dans le cas de la cavité vaginale de la femme par exemple.

Conformément au document WO 80/01353, on a décrit et proposé un dispositif à usage unique de transfert d'un liquide actif dans une cavité intracorporelle, par exemple bronchiale, présentant une raideur intrinsèque suffisante pour permmettre son introduction par poussée. Ce dispositif comprend :
- un élément central
- un élément périphérique tubulaire, disposé autour de l'élément central, et concentrique avec ce dernier,
- des moyens d'appui avec étanchéïté de l'élément périphérique, contre la muqueuse de la cavité intracorporelle ; ces moyens d'appui comprennent un conduit disposé à l'extérieur de l'élément périphérique, susceptible de se déplacer par rapport à ce dernier en translation, et une membrane étanche reliant le bord libre du conduit et celui de l'élément périphérique ; deux positions relatives sont ainsi définies entre l'élément périphérique et le conduit, à savoir une première position dans laquelle l'élément périphérique est rétracté à l'intérieur du conduit et la membrane peut jouer le rôle de diaphragme fermé par insufflation d'un gaz approprié entre l'élément périphérique et le conduit, et une deuxième position dans laquelle l'élément périphérique est sorti par rapport au conduit, et dans laquelle la membrane peut être gonflée pour prendre appui contre la muqueuse de la cavité intracorporelle,
- un moyen temporaire de protection de l'élément central, étanche vis à vis des liquides ; ce moyen temporaire de protection se confond avec le moyen d'appui précité, lorsque l'élément périphérique est disposé à l'intérieur du conduit, avec gonflement de la membrane jouant le rôle de diaphragme,
- une source de liquide actif pouvant être disposée pour communiquer avec l'intérieur de l'élément périphérique.

Conformément au document US-A3519364, il est décrit un dispositif utilisant un liquide actif pour obtenir un échantillon dans une cavité corporelle.

La présente invention a pour objet un dispositif à usage unique, tel que défini précédemment, assurant une absence quasi totale d'écoulement externe, ou du liquide actif et/ou des liquides ou fluides corporels, ou de tout liquide présent dans la cavité intracorporelle considérée, pendant tout le temps où ledit dispositif est présent ou maintenu dans ladite cavité, par simple construction de cette dernière ; à cette fin, selon l'invention, en combinaison :
- l'élément périphérique comprend un tampon agencé pour absorber tout liquide en s'expansant, et en venant de ce fait en appui d'étanchéité contre la muqueuse de la cavité intracorporelle,
- le moyen temporaire de protection étanche l'élément périphérique vis à vis des liquides, et ceci par rapport à l'élément central, lesdits éléments étant disposés pour communiquer l'un avec l'autre dans la cavité intracorporelle, une fois le moyen temporaire de protection retiré,
- et la source du liquide actif est comprise dans l'élément central ou l'élément périphérique, tandis qu'en correspondance un moyen de recueil du liquide actif, ayant circulé dans la cavité intracorporelle est compris dans l'élément périphérique ou l'élément central.

Ce dispositif à usage unique peut être complété par un élément d'application à l'intérieur de la cavité intracorporelle, monté sur l'élément central et/ou l'élément périphérique, de manière solidaire dans le sens de l'introduction dans ladite cavité, et de manière détachable dans le sens inverse.

Aux fins d'un prélèvement et/ou d'une analyse du liquide ou fluide corporel présent dans la cavité intracorporelle considérée, le dispositif peut comprendre en outre :
- un élément de transport du liquide actif ayant circulé dans la cavité intracorporelle, mélangé éventuellement audit liquide ou fluide corporel, ledit élément étant sous une forme allongée, par exemple une cordelette, en continuité d'écoulement à une extrémité avec l'élément central ou l'élément périphérique, respectivement ;
- un élément externe d'analyse extemporanée d'un constituant du liquide ou fluide corporel, mélangé au liquide actif, ou d'une condition biochimique, chimique, ou biologique dudit liquide corporel ; cet élément d'analyse est disposé à l'extrémité libre de l'élément de transport.

Par "constituant" du liquide ou fluide corporel, on entend aussi bien un constituant chimique ou biochimique, qu'un constituant biologique ou vivant, par exemple cellulaire.

A titre d'exemple, s'agissant de la glaire cervicale considérée comme un fluide corporel, et aux fins de détecter les périodes de fertilité chez la femme, le constituant biochimique ou biologique à analyser est une peroxydase ou composé présentant une activité peroxydasique, et l'élément externe d'analyse extemporanée comprend ou forme un réactif ou un système de réactifs, notamment colorés, par exemple un composé d'oxydo-réduction, dont au moins la forme oxydée est colorée, par exemple le gaïacol.

Par "analyse", on entend toute méthode ou dispositif permettant d'identifier, séparer, enrichir, ou quantifier, le constituant ou analyte recherché dans le liquide corporel, ou toute condition de ce dernier, par exemple son acidité ou pH.

La présente invention est maintenant décrite par référence au dessin annexé, dans lequel :
- la figure 1 représente une vue éclatée d'un dispositif selon un premier mode d'exécution de l'invention ;
- la figure 2 représente une vue en perspective du dispositif représenté à la figure 1, prêt à l'emploi ;
- la figure 3 représente une vue en coupe axiale du dispositif représenté aux figures 1 et 2 ;
- la figure 4 représente une vue en coupe des éléments central et périphérique du dispositif représenté aux figures 1 à 3, une fois introduits et maintenus dans une cavité intracorporelle, après avoir éliminé le moyen temporaire de protection de l'élément périphérique ;
- la figure 5 représente une vue en coupe d'un détail du dispositif représenté aux figures 1 à 3 ;
- la figure 6 représente une vue en coupe axiale d'un dispositif selon un deuxième mode d'exécution de l'invention ;
- la figure 7 représente une vue en coupe axiale des éléments axial et périphérique du dispositif représenté à la figure 6, une fois introduit et maintenu dans une cavité intracorporelle, après élimination du moyen temporaire de protection de l'élément périphérique ;
- la figure 8 représente une vue en coupe axiale d'un dispositif selon un troisième mode d'exécution de l'invention ;
- la figure 9 représente une vue en coupe axiale des éléments axial et périphérique du dispositif représenté à la figure 8, une fois introduits et en position dans une cavité intracorporelle ;
- les figures 10 et 11 représentent, respectivement en perspective et en coupe, un dispositif selon un quatrième mode d'exécution de l'invention ;
- les figures 12 et 13 représentent le dispositif montré aux figures 10 et 11, respectivement solidarisé à un élément d'application, et désolidarisé de ce dernier, afin de permettre son détachement ;
- la figure 14 représente en perspective un dispositif selon un cinquième mode d'exécution de l'invention, plus particulièrement destiné et adapté au traitement de lésions pathologiques de la cavité vaginale ;
- la figure 15 représente le dispositif montré à la figure 14, de manière schématique en coupe axiale, et en position dans la cavité vaginale ;
- la figure 16 représente un dispositif selon un sixième mode d'exécution de l'invention, toujours destiné et adapté au traitement de la cavité vaginale ;
- la figure 17 représente un septième mode d'exécution de l'invention, et plus particulièrement un dispositif de traitement de la cavité vaginale ;
- la figure 18 représente de manière schématique un huitième mode d'exécution de l'invention, consistant également en un dispositif de traitement de la cavité vaginale ;
- la figure 19 représente un dispositif selon un neuvième mode d'exécution de l'invention, en coupe axiale, et sans son moyen temporaire de protection de l'élément périphérique.

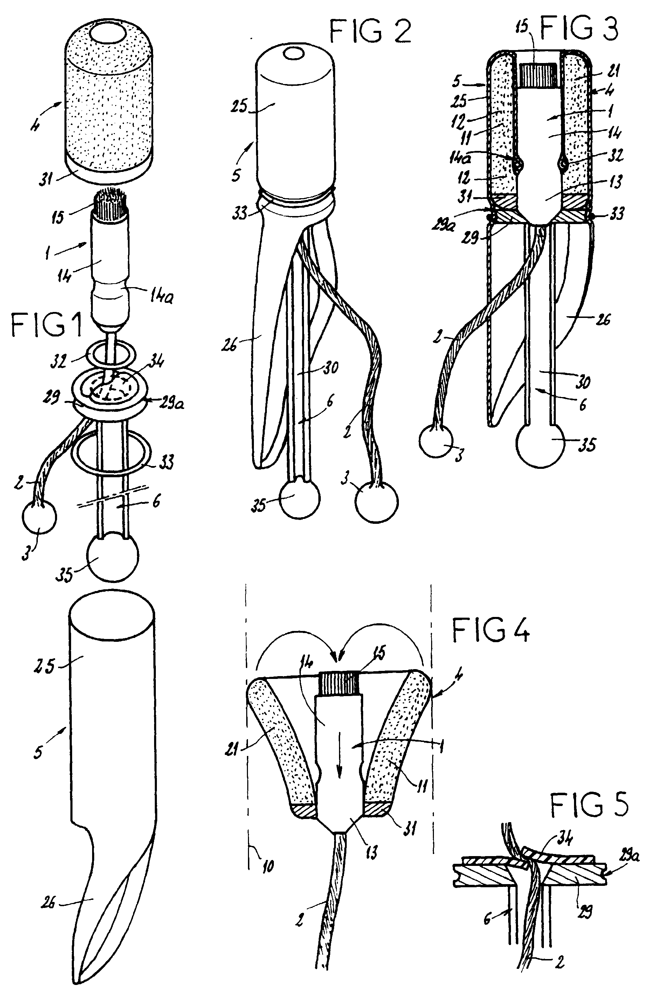

Conformément aux figures 1 à 5, un dispositif selon l'invention, à usage unique, de transfert d'un liquide actif dans une cavité intracorporelle représentée de manière schématique à la figure 4 sous la référence numérique 10, a une forme générale, et présente une raideur intrinsèque suffisante, pour permettre son introduction par poussée, et son maintien dans la cavité 10 par simple constriction de cette dernière.

De manière générale, ce dispositif comprend :
- un élément central 1 comportant des moyens de contact ou de mise en relation avec la muqueuse ou l'intérieur de la cavité intracorporelle 10 ;
- un élément périphérique 4 disposé autour de, et plus précisément de manière concentrique autour de l'élément central ; cet élément comprend un tampon 11 agencé ou choisi pour absorber tout liquide ou fluide en s'expansant, et en venant de ce fait en appui d'étanchéité contre la muqueuse de la cavité intracorporelle 10 ;
- un moyen temporaire 5, étanche vis-à-vis des liquides de protection de l'élément périphérique 4 par rapport à l'élément central 1 ; l'élément central 1 et l'élément périphérique 4 sont disposés, comme montré à la figure 4, pour communiquer l'un avec l'autre dans la cavité intracorporelle 10, une fois le moyen 5 temporaire de protection retiré ;
- une source 12 du liquide actif, comprise dans l'élément périphérique 4 ;
- un moyen de recueil 13 du liquide actif, ayant circulé dans la cavité intracorporelle, et ayant entraîné éventuellement avec lui un fluide ou liquide corporel, ce moyen étant compris dans l'élément central 1 ;
- un élément d'application 6 à l'intérieur de la cavité 10 intracorporelle, monté sur l'élément central 1 et/ou l'élément périphérique 4, de manière solidaire dans le sens de l'introduction dans la cavité 10, et de manière détachable dans le sens inversé ;
- un élément de transport 2, par exemple une mèche de transport du liquide actif ayant circulé dans la cavité intracorporelle 10, de forme allongée, par exemple une cordelette, en continuité à une extrémité avec l'élément central 1, et plus précisément le moyen de recueil 13 ;
- et un élément externe d'analyse 3 extemporanée d'un constituant du liquide corporel présent dans la cavité intracorporelle 10, mélangé au liquide actif, ou d'analyse extemporanée d'une condition biochimique, chimique, ou biologique de ce même liquide corporel ; cet élément d'analyse est disposé à l'extrémité libre de l'élément de transport 2.

L'élément central 1 comprend un noyau 14 pourvu de moyens filiformes 15, notamment des filaments capillaires, de mise en contact avec la muqueuse de la cavité intracorporelle. Ces moyens filiformes sont maintenus en forme par le moyen temporaire 5 de protection décrit ci-après.

Les filaments capillaires 15 peuvent être obtenus en toute matière synthétique ou naturelle, notamment biocompatible, par exemple en fibres absorbantes naturelles (coton, lin, etc...).

L'élément périphérique 4 comprend un manchon 21 d'un matériau susceptible d'absorber un liquide ou fluide en s'expansant, et entourant le noyau 14 de l'élément central 1, pourvu d'éléments filiformes 15.

Le matériau absorbant du manchon 21 est expansible et/ou spongieux, et consiste par exemple en des filaments, des fibres capillaires, ou des fibres naturelles, etc...

Ce manchon a une forme cylindrique lorsqu'il est maintenu par le moyen temporaire 5 de protection décrit ci-après, et évolue vers une forme en corolle, une fois le moyen de protection temporaire 5 enlevé, et le dispositif selon l'invention introduit et maintenu de manière relativement bloquée dans la cavité intracorporelle (cf figure 4).

Le matériau absorbant du manchon 21 est imprégné à saturation du liquide actif, par exemple un sérum physiologique salé, et/ou d'un agent fluidifiant non agressif, et/ou d'un agent thérapeutique à diffusion locale. Il est maintenu dans une conformation non expansée par le moyen temporaire de protection 5 décrit ci-après. C'est dans l'orifice central cylindrique du manchon 21 qu'est disposé l'élément central 1 précédemment décrit. La partie inférieure 31 du manchon 21 est imperméable, soit par imprégnation avec un produit approprié du matériau absorbant, soit en rapportant une plaque imperméable.

Le moyen 5 temporaire de protection comprend une gaine 25 entourant ou emballant au moins partiellement, de manière étanche, l'élément périphérique 4, tout autour de l'élément central 1. Cette gaine comporte ou est solidaire à une extrémité d'un appendice 26, par exemple une boucle de traction, permettant d'extraire la gaine 25, pour exposer l'élément périphérique 4 dans la cavité intracorporelle 10.

A l'exception de la partie en boucle ou appendice 26, cette gaine 25 a la forme générale d'une chaussette partiellement retournée sur elle-même et autour du manchon 21. La bordure libre intérieure de la gaine 25 est maintenue en contact étanche dans une gorge circulaire 14a du noyau 14, au moyen d'un joint tangue 32, ou d'une soudure facilement déchirable. De la même manière, la zone annulaire de transition entre la partie en chaussette et la partie en boucle 26 de la gaine 25 est serrée en contact étanche, dans une gorge 29a de l'élément d'appui 29 décrit ci-après de l'élément d'application 6, au moyen d'un joint torique 33, ou par une soudure facilement déchirable, mais suffisamment résistante pour maintenir l'étanchéité à l'intérieur de la partie en chaussette de la gaine 25. Eventuellement, la gaine 25 peut être suffisamment serrée sur l'élément périphérique 4, ou plus précisément sur la base 31 imperméable du manchon 21, toujours de manière étanche.

La gaine 25 est réalisée en un matériau, notamment biocompatible, par exemple une matière plastique thermo-rétractable, imperméable. Elle est très fine, mais suffisamment résistante pour permettre, en tirant sur la boucle 26, comme décrit ci-après, de rompre les liaisons étanches au niveau des gorges 14a, 29a, et éventuellement de la base 31, puis déplier la partie de l'enveloppe en forme de chaussette, et la séparer ou l'extraire du dispositif, en libérant l'élément périphérique 4, et l'exposant au sein de la cavité intracorporelle 10.

Par ailleurs, la gaine 25, de forme générale cylindrique, est suffisamment souple et fine, ainsi que lisse et glissante sur sa face externe, pour faciliter l'introduction du dispositif dans la cavité intracorporelle.

L'élément de transport 2 sert à la fois à diriger le liquide actif , et éventuellement le ou les liquides corporels prélevés au moyen de ce dernier, jusqu'à l'élément d'analyse 3. Il sert aussi à retirer la totalité du dispositif selon l'invention après usage. L'élément de transport 2 a un diamètre suffisamment fin pour ne pas constituer une gène ou un inconfort dans la cavité corporelle. Cet élément peut être unique ou double, et se présenter sous la forme d'un gros fil ou d'une cordelette par exemple.

L'élément d'analyse 3 est en continuité d'écoulement avec l'élément de transport 2. Cet élément 3 supporte des réactifs appropriés, déposés à cet effet. Avantageusement, cet élément d'analyse 3 a une forme sphérique. Toutefois, dans le cas ou cet élément ne forme pas une petite sphère ou un cylindre, les réactifs sont déposés à l'extrémité libre de la cordelette, ou à la partie extériorisée d'une cordelette double.

L'élément d'application 6 comprend un élément d'appui 29, par exemple un disque, fixé à l'extrémité d'une tige axiale 30, par exemple un tube évidé diamétralement. L'élément central 1 est monté de manière étanche à une extrémité, contre et dans un orifice adapté du disque 29, le tout de manière alignée avec la tige axiale 30. Comme montré plus particulièrement à la figure 5, le passage étanche de l'élément de transport 6, dans l'orifice ménagé sur le disque 29 est assuré au moyen d'un joint élastique 34. La tige axiale 30, ayant la forme d'un tube fendu, est adaptée pour le passage de l'élément de transport 2. La tige axiale 30 est terminée par un renflement 35 afin de faciliter la manipulation et le retrait de l'élément d'application 6.

L'utilisation ou mise en oeuvre du dispositif précédemment décrit s'effectue de la manière suivante, en partant de la conformation représentée à la figure 2 :
- en poussant sur l'élément d'application 6, le dispositif est introduit dans la cavité intracorporelle 10, laquelle le maintient en position par simple constriction ;
- en tirant sur la boucle 26, on libère la gaine 25, ou moyen temporaire de protection 5, par rapport à l'élément central 1 et l'élément périphérique 4, on développe ou déplie la gaine 25, et on l'extrait de la cavité intracorporelle, en glissant le long de l'élément périphérique 4 ;
- l'élément périphérique 4 est libéré et s'expanse alors, en contact relativement étanche avec la cavité intracorporelle 10, comme montré à la figure 4, ce qui assure aussi son maintien en place ;
- dès ce moment, le liquide actif est libéré dans la cavité 10, et entraîne avec lui le liquide corporel présent dans la cavité 10, le tout vers l'élément central 1, par l'intermédiaire des moyens filiformes 15 et du moyen de recueil 13, le tout vers l'élément d'analyse 3, qui peut être observé ou visualisé par l'utilisateur, à l'extérieur de la cavité 10 ;
- une fois la réaction ou l'absence de réaction mise en évidence, en tirant sur l'élément de transport 2, on peut extraire le dispositif, puis le jeter.

Le dispositif précédemment décrit apporte en outre les avantages déterminants suivants :
- il est d'une utilisation particulièrement simple, sûre et efficace ;
- les produits chimiques et/ou biologiques nécessaires à l'analyse étant reportés à l'extérieur de la cavité intracorporelle, il ne peut exister aucune irritation, voire brûlure, au contact des tissus vivants, et plus particulièrement au niveau des muqueuses de la cavité intracorporelle ;
- les résultats d'analyse peuvent être lus directement sans manipulation, ni contact direct avec les produits chimiques ou biologiques de réaction.

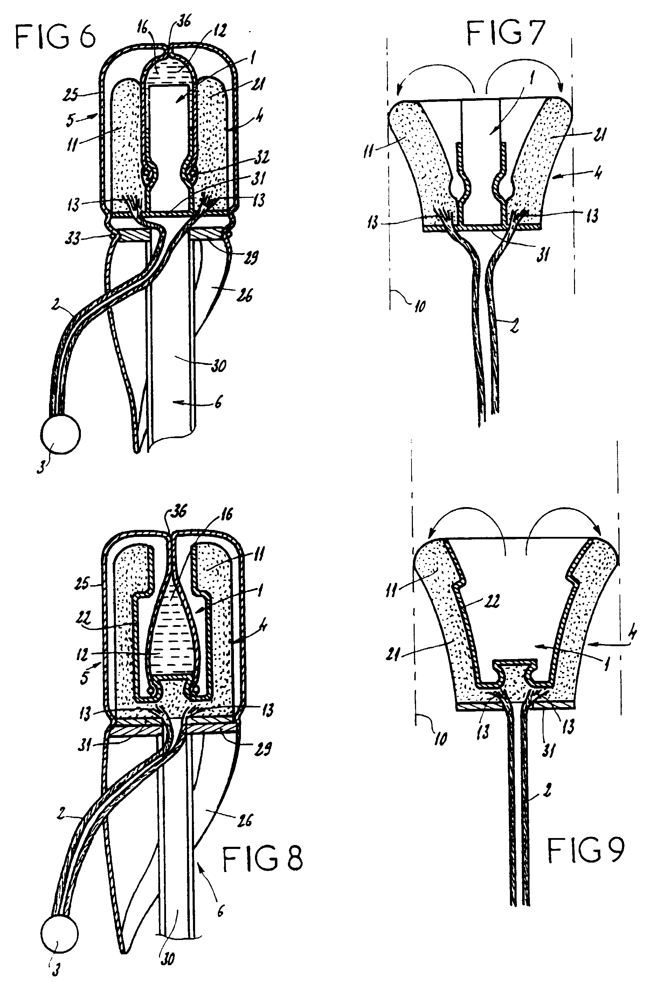

Le dispositif représenté aux figures 6 et 7 diffère du dispositif représenté aux figures 1 à 5, par les caractéristiques suivantes :
- à l'inverse du dispositif décrit précédemment, la source 12 du liquide actif est comprise dans l'élément central 1, et le moyen de recueil 13 du liquide actif, ayant circulé dans la cavité intracorporelle 10, est compris dans l'élément périphérique 4 ; en correspondance, l'élément de transport 2 est en continuité à une extrémité avec l'élément périphérique 4 ;
- l'élément central 1 comprend ou consiste en une cavité 16 contenant le liquide actif, cloisonnée ou déterminée par la gaine 25, refermée sur elle-même par un joint ou une soudure 36.

Le dispositif représenté aux figures 8 et 9 diffère de celui représenté aux figures 6 et 7, par le fait qu'une cloison 22 d'étanchéité est ménagée entre l'élément central 1 et l'élément périphérique 4.

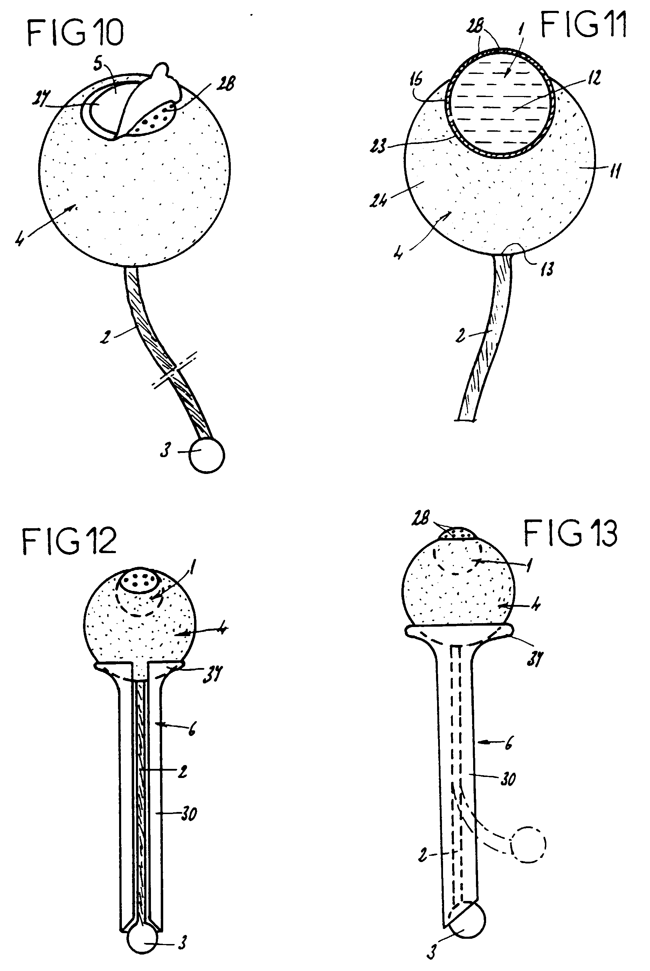

Le dispositif représenté par référence aux figures 10 à 13 se différencie du dispositif représenté par référence aux figures 1 à 5, par les caractéristiques techniques suivantes. L'élément périphérique 4 entoure au moins partiellement l'élément central 1, ayant la forme d'une cavité 16, elle-même constituée par un matériau 23 élastique et souple, par exemple en caoutchouc. L'élément périphérique 4 est quant à lui constitué par un matériau 24 susceptible d'absorber un liquide en s'expansant, par exemple un matériau à base de fibres tel que décrit précédemment.

Le moyen temporaire de protection 5 consiste en une capsule 27, isolant temporairement des trous 28 de passage du liquide actif, vis-à-vis de l'élément périphérique 4.

L'élément d'application 6 comprend une collerette 37, fixée à l'extrémité de la tige axiale 30, de manière à permettre le logement de l'élément de transport 2, en extension entre l'élément périphérique 4 maintenu contre ladite collerette et l'élément d'analyse 3 maintenu à l'autre extrémité de la tige axiale 30, constitué comme précédemment par un tube de section longitudinale ouverte.

La capsule 27 est facilement décollable, pour libérer les trous 28, au moment de l'utilisation, c'est-à-dire une fois le dispositif mis en place dans la cavité intracorporelle. Dès ce moment, toute pression exercée sur la cavité 16 permet sa vidange, en sorte que le liquide actif imprègne l'élément périphérique 4 qui, en se gorgeant dudit liquide, comprime à son tour la cavité 16 pour finir d'en évacuer le contenu.

Lorsque la cavité 16 a été partiellement vidée, l'élément d'analyse 3 est poussé en avant du tube 30 pour dégager l'élément de transport 2, et pouvoir détacher et extraire l'élément d'application 6 de la cavité intracorporelle. Ensuite, comme pour les dispositifs précédents, il suffit de tirer sur l'élément de transport 2 pour extraire l'ensemble constitué par l'élément central 1 ou cavité 16 et l'élément périphérique 4 en forme de sphère.

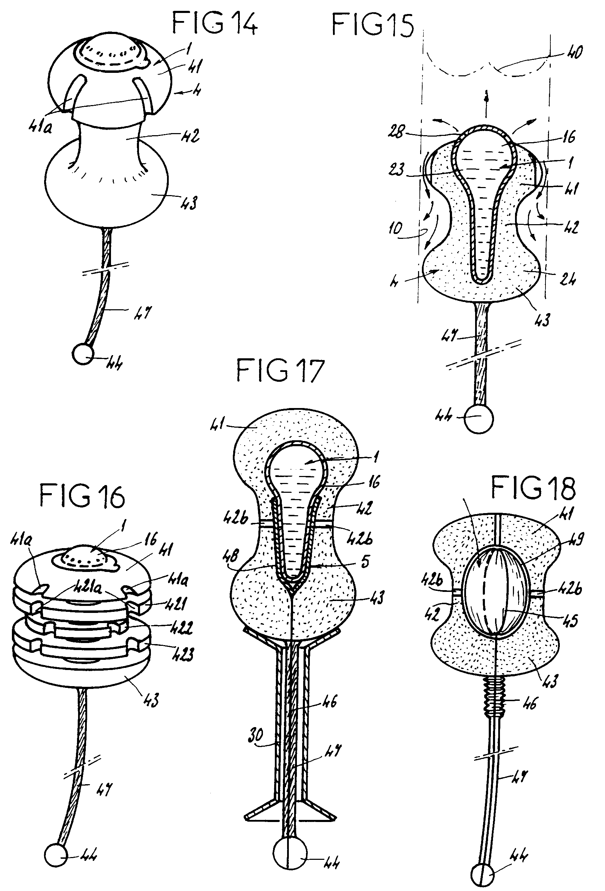

Conformément aux figures 14 à 18, on décrit maintenant différents dispositifs selon l'invention pour le traitement de lésions pathologiques de la cavité vaginale. A cette fin, le liquide actif est un liquide thérapeutique, pouvant contenir des produits antibiotiques, antiseptiques, des dérivés hormonaux, des corticoïdes, différents agents adoucissants, trophiques locaux, etc...

Conformément aux figures 14 et 15, et par différence aux modes d'exécution représentés et décrits précédemment par référence aux figures 10 à 13, l'élément périphérique 4 comprend deux renflements 41 et 43, de part et d'autre d'un noyau central 42, de section rétrécie, et a donc généralement la forme d'une "bobine de fil". La cavité 16 contenant le liquide actif est disposée principalement dans le renflement supérieur 41, mais aussi pour une autre partie à l'intérieur et au centre du noyau central 42.

Le renflement 41 dans lequel est disposée la cavité 16, comporte différentes rainures 41a d'écoulement du liquide actif vers le noyau central, au contact de la paroi vaginale lorsque le dispositif est en place dans la cavité vaginale 10 et en butée contre le col cervical 40. Et ce liquide actif, éventuellement mélangé au flux vaginal, est finalement recueilli dans l'autre renflement 43 de l'élément périphérique 4.

Le dispositif comporte un lien 47 d'extraction de la cavité vaginale 10, solidaire à une extrémité de l'élément périphérique 4, et plus précisément de l'autre renflement 43, et comprenant à l'autre extrémité un moyen de préhension 44.

Le dispositif précédemment décrit s'avère bien adapté pour un contact préférentiel avec le col cervical, comme représenté à la figure 15.

Le noyau central 42, de section rétrécie ou non, permet, grâce aux rainures 41a, au liquide actif d'atteindre les parois vaginales. Ce liquide est finalement absorbé dans l'autre renflement 43 de l'élément périphérique 4, éventuellement avec tout liquide suintant dans la cavité vaginale, provenant par exemple d'un écoulement pathologique, ou d'une réaction biologique déclenchée par le liquide actif médicamenteux. L'autre renflement peut d'ailleurs être imprégné avec tout réactif coloré approprié, permettant par exemple de détecter les toxines des "germes" détruits par le liquide actif. C'est plus précisément ledit autre renflement 43 qui permet d'éviter tout écoulement important à l'intérieur de la cavité vaginale, lors du traitement de cette dernière avec un dispositif selon l'invention.

Le dispositif représenté à la figure 16 diffère de celui représenté aux figures 14 et 15, par le fait que :
- le noyau central 42 a approximativement la même section que les renflements 41 et 43, et comporte une pluralité de tranches 421 à 423, séparées et entretoisées les unes par rapport aux autres ;
- ces tranches 421 a 423 comportent des encoches, par exemple 421a, en correspondance les unes avec les autres et avec une nervure 41a d'écoulement du liquide actif.

Le dispositif représenté à la figure 17 diffère de celui représenté par référence aux figures 14 et 15, par le fait que, d'une part le noyau central 42 de l'élément pé riphérique 4 comprend des orifices 42b de passage du liquide actif vers l'extérieur et la paroi vaginale, et d'autre part, le moyen temporaire de protection 5 comprend un moyen amovible 48 d'étanchéité entre, d'un côté l'intérieur de la cavité 16, et de l'autre côté les orifices 42b précités. Préférentiellement, ce moyen amovible est une gaine d'isolement 48 pouvant être retirée séparément par tirage d'un fil 46, à partir de l'extérieur de la cavité vaginale.

Le dispositif représenté à la figure 18 diffère de celui représenté à la figure 17, par le fait que le moyen amovible d'étanchéité, entre l'intérieur de la cavité 16 et les orifices 42b de passage du liquide actif, comprend une enveloppe 49 délimitant la cavité 16, et des moyens 45 agencés pour rompre l'enveloppe 49, et être notamment activés 46 en tirant sur le lien 47 d'extraction.

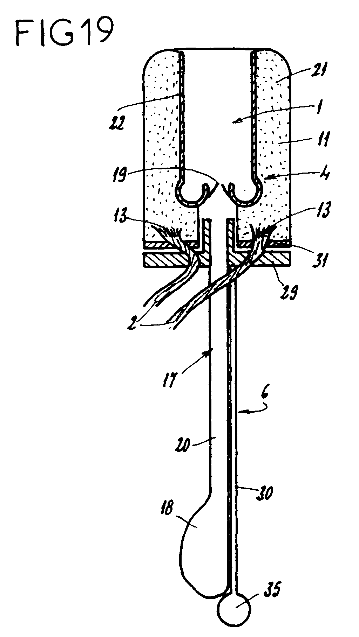

Le dispositif représenté à la figure 19 diffère de celui représenté aux figures 1 à 5, par le fait que l'élément central 1 comprend un moyen 17 d'introduction contrôlée du liquide actif, à partir de l'extérieur du dispositif. Ce moyen 17 comporte un réservoir 18 souple et dépressible, disposé du côté de l'extrémité 35 de la tige axiale 30, une valve 19 souple, disposée à l'entrée de l'élément central 1 ayant la forme d'une cavité délimitée par la cloison d'étanchéité 22, et une canalisation disposée entre le réservoir 18 et la valve 19. La valve 19 est agencée en sorte de s'ouvrir dans le sens de l'introduction du liquide actif, et de se refermer dans l'autre sens.

## Revendications

1. Dispositif à usage unique de transfert d'un liquide actif dans une cavité intracorporelle (10), présentant une raideur intrinsèque suffisante, pour permettre son introduction par poussée, ledit dispositif comprenant :
- un élément central (1) ;
- un élément périphérique (4) disposé autour de l'élément central (1), notamment concentrique avec ce dernier ;
- des moyens (11) d'appui avec étanchéité, de l'élément périphérique (4), contre la muqueuse de la cavité intracorporelle (10) ;
- un moyen temporaire de protection (5), étanche vis-à-vis des liquides, de l'élément central (1) ;
- une source (12) du liquide actif,
caractérisé en ce que l'élément périphérique (4) comprend un tampon (11) agencé pour absorber tout liquide en s'expansant, et en venant de ce fait en appui d'étanchéité contre la muqueuse de ladite cavité intracorporelle (10), le moyen temporaire de protection (5) étanche, l'élément périphérique(4), vis à vis des liquides par rapport à l'élément central (1), lesdits éléments étant disposés pour communiquer l'un avec l'autre, dans ladite cavité intracorporelle, une fois le moyen temporaire de protection retiré, et la source (12) du liquide actif est comprise dans l'élément central (1) ou l'élément périphérique (4), tandis qu'en correspondance un moyen de reçueil (13) du liquide actif, ayant circulé dans la cavité intracorporelle est, compris dans l'élément périphérique (4) ou l'élément central(1) ;

2. Dispositif selon la revendication 1 caractérisé en ce que l'élément central (1) comporte des moyens (15) de contact avec la muqueuse ou l'intérieur de la cavité intracorporelle (10).

3. Dispositif selon la revendication 1, caractérisé en ce que la source (12) du liquide actif est comprise dans l'élément périphérique (4), et le moyen de recueil (13) du liquide actif, ayant circulé dans la cavité intracorporelle (10) est compris dans l'élément central (1).

4. Dispositif selon la revendication 1, caractérisé en ce que la source (12) du liquide actif est comprise dans l'élément central (1), et le moyen de recueil (13) du liquide actif, ayant circulé dans la cavité intracorporelle (10), est compris dans l'élément périphérique (4).

5. Dispositif selon la revendication 2, caractérisé en ce que l'élément central (1) comprend un noyau (14) pourvu de moyens filiformes (15), notamment filaments capillaires, de mise en contact avec la muqueuse de la cavité intracorporelle (10), lesdits moyens filiformes étant notamment maintenus en forme par le moyen temporaire (5) de protection.

6. Dispositif selon les revendications 1 et 4, caractérisé en ce que l'élément central (1) comprend une cavité (16) contenant le liquide actif.

7. Dispositif selon les revendications 1 et 4, caractérisé en ce que l'élément central (1) comprend un moyen (17) d'introduction contrôlée du liquide actif, à partir de l'extérieur dudit dispositif, notamment un réservoir (18) souple et dépressible, pourvu d'une valve (19) souple, coopérant avec une canalisation (20) disposée selon l'axe dudit dispositif.

8. Dispositif selon la revendication 5, caractérisé en ce que l'élément périphérique (4) comprend un manchon (21) d'un matériau susceptible d'absorber un liquide en s'expansant, entourant le noyau (14) pourvu d'éléments filiformes (15).

9. Dispositif selon la revendication 6, caractérisé en ce qu'une cloison (22) d'étanchéité est ménagée entre l'élément central (1) et l'élément périphérique (4).

10. Dispositif selon la revendication 6, caractérisé en ce que l'élément périphérique (4) entoure au moins partiellement la cavité (16), elle-même constituée par un matériau (23) élastique et souple, et ledit élément périphérique est constitué par un matériau (24) susceptible d'absorber un liquide en s'expansant.

11. Dispositif selon la revendication 1, caractérisé en ce que le moyen (5) temporaire de protection comprend une gaine (25) emballant au moins partiellement de manière étanche l'élément périphérique (4), tout autour de l'élément central (1), et comporte un appendice (26), par exemple une boucle, de traction et d'exposition dudit élément périphérique dans la cavité intracorporelle (10).

12. Dispositif selon les revendications 6 et 11, caractérisé en ce que la gaine (25) cloisonne également la cavité (16) du liquide actif.

13. Dispositif selon la revendication 10, caractérisé en ce que le moyen temporaire de protection (5) comprend une capsule (27) isolant temporairement des trous (28) de passage du liquide actif, vis-à-vis de l'élément périphérique (4).

14. Dispositif selon la revendication 1, caractérisé en ce qu'il comprend un élément d'application (6), à l'intérieur de la cavité (10) intracorporelle, monté sur l'élément central (1) et/ou l'élément périphérique (4), de manière solidaire dans le sens de l'introduction dans ladite cavité, et de manière détachable dans le sens inverse.

15. Dispositif selon la revendication 14, caractérisé en ce que l'élément d'application (6) comprend un élément d'appui (29), par exemple un disque ou une collerette (37) fixé à l'extrémité d'une tige axiale (30), par exemple un tube.

16. Dispositif selon la revendication 15, caractérisé en ce que l'élément central (1) est monté de manière étanche à une extrémité sur un disque (29), de manière alignée avec la tige axiale (30).

17. Dispositif selon la revendication 1, caractérisé en ce qu'il comprend un élément de transport (2) du liquide actif, ayant circulé dans la cavité intracorporelle (10), de forme allongée, par exemple une cordelette, en continuité à une extrémité avec l'élément central (1), ou l'élément périphérique (4).

18. Dispositif selon la revendication 17, caractérisé en ce qu'il comprend un élément externe d'analyse (3) extemporanée d'un constituant d'un liquide corporel présent dans la cavité intracorporelle (10), mélangé au liquide actif, ou d'une condition biochimique, chimique, ou biologique dudit liquide corporel, ledit élément d'analyse étant disposé à l'extrémité libre de l'élément de transport (2).

19. Dispositif selon les revendications 15 et 17, caractérisé en ce que la tige axiale (30) est adaptée pour le passage de l'élément de transport (2).

20. Dispositif selon la revendication 1, caractérisé en ce que le liquide actif comprend au moins l'un quelconque des produits suivants, à savoir un agent liquide de mouillage, solubilisant, ou fluidifiant, un agent thérapeutique, prophylactique ou de diagnostic, un agent cosmétique ou d'hygiène corporelle, et un agent antiseptique, bactéricide, fongicide, ou spermicide.

## Claims

1. Disposable device for transferring an active liquid into an intracorporeal cavity (10), which presents sufficient intrinsic stiffness to allow it to be introduced by pushing, the said device comprising,
- a central element (1);
- a peripheral element (4) arranged around the central element (1), in particular concentric with the latter;
- means (11) for applying, without leaks, the peripheral element (4) against the mucosa of the intracorporeal cavity (10);
- liquid-tight means (5) for temporary protection of the central element (1);
- a source (12) of the active liquid,
characterized in that the peripheral element (4) comprises a pad (11) which is designed to absorb any liquid by expanding, and therefore coming into leaktight contact against the mucosa of the said intracorporeal cavity (10), the temporary protection means (5) seals the peripheral element (4) against liquids with respect to the central element (1), the said elements being arranged so as to communicate with one another, in the said intracorporeal cavity, once the temporary protection means has been removed, and the source (12) of the active liquid is contained in the central element (1) or the peripheral element (4), while a means (13) for collecting the active liquid which has flowed through the intracorporeal cavity is correspondingly contained in the peripheral element (4) or the central element (1)

2. Device according to Claim 1, characterized in that the central element (1) includes means (15) for contact with the mucosa or the interior of the intracorporeal cavity (10).

3. Device according to Claim 1, characterized in that the source (12) of the active liquid is contained in the peripheral element (4), and the means (13) for collecting the active liquid which has flowed through the intracorporeal cavity (10) is contained in the central element (1).

4. Device according to Claim 1, characterized in that the source (12) of the active liquid is contained in the central element (1), and the means (13) for collecting the active liquid which has flowed through the intracorporeal cavity (10) is contained in the peripheral element (4).

5. Device according to Claim 2, characterized in that the central element (1) comprises a core (14) provided with filamentous means (15), in particular hair filaments, for contact with the mucosa of the intracorporeal cavity (10), the said filamentous means being, in particular, held in shape by the temporary protection means (5).

6. Device according to Claims 1 and 4, characterized in that the central element (1) comprises a cavity (16) containing the active liquid.

7. Device according to Claims 1 and 4, characterized in that the central element (1) comprises a means (17) for controlled introduction of the active liquid, from outside the said device, in particular a flexible depressible reservoir (18), provided with a flexible valve (19), co-operating with a duct (20) arranged along the axis of the said device.

8. Device according to Claim 5, characterized in that the peripheral element (4) comprises a sleeve (21) made of a material capable of absorbing a liquid by expanding, surrounding the core (14) provided with filamentous elements (15).

9. Device according to Claim 6, characterized in that a sealing partition (22) is formed between the central element (1) and the peripheral element (4).

10. Device according to Claim 6, characterized in that the peripheral element (4) at least partially surrounds the cavity (16), which itself consists of an elastic flexible material (23), and the said peripheral element consists of a material (24) capable of absorbing a liquid by expanding.

11. Device according to Claim 1, characterized in that the temporary protection means (5) comprises a sheath (25) wrapping the peripheral element (4) at least partially without leaks, all around the central element (1), and includes an appendage (26), for example a loop, for pulling and exposing the said peripheral element in the intracorporeal cavity (10).

12. Device according to Claims 6 and 11, characterized in that the sheath (25) also partitions the cavity (16) of the active liquid.

13. Device according to Claim 10, characterized in that the temporary protection means (5) comprises a capsule (27) temporarily isolating passage holes (28) for the active liquid from the peripheral element (4).

14. Device according to Claim 1, characterized in that it comprises an element (6) for application, inside the intracorporeal cavity (10), mounted on the central element (1) and/or the peripheral element (4), secured in the direction of introduction into the said cavity, and detachable in the opposite direction.

15. Device according to Claim 14, characterized in that the application element (6) comprises a bearing element (29), for example a disc or a collar (37) fixed to the end of an axial rod (30), for example a tube.

16. Device according to Claim 15, characterized in that the central element (1) is mounted without leaks at one end on a disc (29), aligned with the axial rod (30).

17. Device according to Claim 1, characterized in that it comprises an element (2) for transporting the active liquid which has flowed through the intracorporeal cavity (10), of elongate shape, for example a cord, in continuity at one end with the central element (1), or the peripheral element (4).

18. Device according to Claim 17, characterized in that it comprises an external element (3) for extemporaneous analysis of a constituent of a body liquid present in the intracorporeal cavity (10), mixed with the active liquid, or a biochemical, chemical or biological condition of the said body liquid, the said analysis element being arranged at the free end of the transport element (2).

19. Device according to Claims 15 and 17, characterized in that the axial rod (30) is designed for the transport element (2) to pass through.

20. Device according to Claim 1, characterized in that the active liquid comprises at least any one of the following products, namely a liquid agent for wetting, solution or fluidizing, a therapeutic, prophylactic or diagnostic agent, a cosmetic or body-hygiene agent, and an antiseptic, bactericidal, fungicidal or spermicidal agent.

## Patentansprüche

1. Vorrichtung für den einmaligen Gebrauch zum Überbringen einer aktiven Flüssigkeit in eine Körperhöhle (10), mit einer ausreichenden eigenen Steifheit, um durch Schieben eingeführt werden zu können, wobei die Vorrichtung folgendes aufweist:
- ein mittiges Element (1);
- ein umfängliches Element (4), das um das mittige Element (1) herum, insbesondere konzentrisch dazu angeordnet ist;
- Mittel (11) zum dichten Anlegen des umfänglichen Elements (4) gegen die Schleimhaut der Körperhöhle (10);
- ein, gegenüber Flüssigkeiten dichtes, zeitweilig vorhandenes Schutzmittel (5) für das mittige Element (1);
- eine Quelle (12) an aktiver Flüssigkeit,
dadurch gekennzeichnet, daß das umfängliche Element (4) einen Tampon (11) aufweist, der derart ausgestaltet ist, daß er jegliche Flüssigkeit absorbiert, sich dabei ausdehnt und aufgrund dieser Tatsache in dichtender Anlage gegen die Schleimhaut der Körperhöhle (10) kommt, daß das zeitweilig vorhandene Schutzmittel (5) das umfängliche Element (4) gegenüber Flüssigkeiten im Verhältnis zum mittigen Element (1) abdichtet, wobei die genannten Elemente derart angeordnet sind, daß sie in der Körperhöhle untereinander in Verbindung stehen, wenn das zeitweilig vorhandene Schutzmittel abgezogen ist, und daß die Quelle (12) an aktiver Flüssigkeit entweder im mittigen Element (1) oder im umfänglichen Element (4) enthalten ist, wohingegen dementsprechend Auffangmittel (13) für die in der Körperhöhle zirkulierten aktiven Flüssigkeit entweder im umfänglichen Element (4) oder im mittigen Element (1) angeordnet sind.

2. Vorrichtung nach Anspruch 1, dadurch gekennzeichnet, daß das mittige Element (1) Mittel (15) aufweist, die in Berührung mit der Schleimhaut oder dem Inneren der Körperhöhle (10) stehen.

3. Vorrichtung nach Anspruch 1, dadurch gekennzeichnet, daß die Quelle (12) an aktiver Flüssigkeit im umfänglichen Element (4) enthalten ist, und daß die Auffangmittel (13) für die aktive Flüssigkeit, die in der Körperhöhle (10) zirkuliert ist, im mittigen Element (1) enthalten sind.

4. Vorrichtung nach Anspruch 1, dadurch gekennzeichnet, daß die Quelle (12) an aktiver Flüssigkeit im mittigen Element (1) enthalten ist, und daß die Auffangmittel (13) für die aktive Flüssigkeit, die in der Körperhöhle (10) zirkuliert ist, im umfänglichen Element (4) enthalten sind.

5. Vorrichtung nach Anspruch 2, dadurch gekennzeichnet, daß das mittige Element (1) einen Kern (14) aufweist, der mit fadenförmigen Mitteln (15), insbesondere mit kapillaren Fasern versehen ist, die mit der Schleimhaut der Körperhöhle (10) in Berührung treten, wobei die fadenförmigen Mittel durch das zeitweilig vorhandene Schutzmittel (5) in Form gehalten sind.

6. Vorrichtung nach den Ansprüchen 1 und 4, dadurch gekennzeichnet, daß das mittige Element (1) einen Hohlraum (16) aufweist, der die aktive Flüssigkeit enthält.

7. Vorrichtung nach den Ansprüchen 1 und 4, dadurch gekennzeichnet, daß das mittige Element (1) Mittel (17) zum kontrollierten Einführen der aktiven Flüssigkeit von der Außenseite der Vorrichtung her aufweist, insbesondere ein flexibles und zusammendrückbares Reservoir (18), das mit einem flexiblen Ventil (19) versehen ist, das mit einer Leitung (20) zusammenwirkt, die längs der Achse der Vorrichtung angeordnet ist.

8. Vorrichtung nach Anspruch 5, dadurch gekennzeichnet, daß das umfängliche Element (4) eine Manschette (21) aus einem solchen Material aufweist, das eine Flüssigkeit absorbiert und sich dabei ausdehnt, wobei diese den Kern (14), der mit den fadenförmigen Elementen (15) versehen ist, umrundet.

9. Vorrichtung nach Anspruch 6, dadurch gekennzeichnet, daß zwischen dem mittigen Element (1) und dem umfänglichen Element (4) eine dichtende Wand (22) angeordnet ist.

10. Vorrichtung nach Anspruch 6, dadurch gekennzeichnet, daß das umfängliche Element (4) zumindest teilweise den Hohlraum (16) umgibt, der wiederum aus einem elastischen und biegsamen Material (23) gebildet ist, und daß das umfängliche Element aus einem Material (24) gebildet ist, das eine Flüssigkeit absorbieren kann und sich dabei ausdehnt.

11. Vorrichtung nach Anspruch 1, dadurch gekennzeichnet, daß das zeitweilig vorhandene Schutzmittel (5) eine Hülle (25) aufweist, die zumindest teilweise dichtend das umfängliche Element (4) um das ganze mittige Element (1) herum umhüllt, und die ein Anhängstück (26), beispielsweise eine Schlaufe zum Ziehen und Freisetzen des umfänglichen Elementes in der Körperhöhle (10) aufweist.

12. Vorrichtung nach den Ansprüchen 6 und 11, dadurch gekennzeichnet, daß die Hülle (25) auch den Hohlraum (16) an aktiver Flüssigkeit abteilt.

13. Vorrichtung nach Anspruch 10, dadurch gekennzeichnet, daß das zeitweilig vorhandene Schutzmittel (5) einen Verschluß (27) aufweist, der zeitweilig Löcher (28) für den Durchtritt der aktiven Flüssigkeit gegenüber dem umfänglichen Element (4) isoliert.

14. Vorrichtung nach Anspruch 1, dadurch gekennzeichnet, daß ein Applikationselement (6) vorgesehen ist, das im Innenraum der Körperhöhle (10) auf dem mittigen Element (1) und/oder dem umfänglichen Element (4) befestigt ist, und zwar in Einführrichtung in die Höhle fest und in entgegengesetzter Richtung lösbar.

15. Vorrichtung nach Anspruch 14, dadurch gekennzeichnet, daß das Applikationselement (6) ein Stützelement (29), beispielsweise eine Scheibe oder einen Kragen (37) aufweist, welches am Ende eines axialen Stabes (30), beispielsweise einem Rohr befestigt ist.

16. Vorrichtung nach Anspruch 15, dadurch gekennzeichnet, daß das mittige Element (1) dichtend an einem Ende auf der Scheibe (29) befestigt ist, und zwar in Ausrichtung mit dem axialen Stab (30).

17. Vorrichtung nach Anspruch 1, dadurch gekennzeichnet, daß es ein Transportelement (2) für die aktive Flüssigkeit, die in der Körperhöhle (10) zirkuliert ist, aufweist und zwar in langerstreckter Form, beispielsweise eine Schnur, die sich an einem Ende vom mittigen Element (1) oder dem umfänglichen Element (4) fortsetzt.

18. Vorrichtung nach Anspruch 17, dadurch gekennzeichnet, daß diese ein außenliegendes Analyseelement (3) für eine vor Ort Analyse eines Bestandteiles einer Körperflüssigkeit aufweist, die in der Körperhöhle (10) vorhanden ist und die mit der aktiven Flüssigkeit vermischt ist, oder eines biochemischen, chemischen oder biologischen Zustandes der Körperflüssigkeit, wobei das Analyseelement am freien äußeren Ende des Transportelementes (2) angeordnet ist.

19. Vorrichtung nach den Ansprüchen 15 und 17, dadurch gekennzeichnet, daß der axiale Stab (30) für den Durchtritt des Transportelementes (2) angepaßt ist.

20. Vorrichtung nach Anspruch 1, dadurch gekennzeichnet, daß die aktive Flüssigkeit zumindest eines der folgenden Produkte aufweist, nämlich ein flüssiges Agens zum Befeuchten, Lösen oder Verflüssigen, ein therapeutisches, prophylaktisches oder diagnostisches Agens, ein kosmetisches oder körperhygienisches Agens und ein antiseptisches, bakterizides, fungizides oder spermizides Agens.
